(19) Europäisches Patentamt
European Patent Office
Office européen des brevets

(11) Publication number: **0 023 078**
**B1**

(12) # EUROPEAN PATENT SPECIFICATION

(45) Date of publication of patent specification: 18.04.84

(21) Application number: 80301811.8

(22) Date of filing: 30.05.80

(51) Int. Cl.[3]: C 07 D 337/14, A 61 K 31/38 //C07C149/40, C07C149/34, C07C149/36, C07C149/415, C07C57/32

(54) **Substituted dibenzo(b,f)thiepin-3-carboxylic acid derivatives, their preparation and pharmaceutical compositions containing them.**

(30) Priority: 01.06.79 US 44445
01.06.79 US 44444

(43) Date of publication of application:
28.01.81 Bulletin 81/4

(45) Publication of the grant of the patent:
18.04.84 Bulletin 84/16

(84) Designated Contracting States:
AT BE CH DE FR GB IT LI LU NL SE

(56) References cited:
EP - A - 0 000 978
EP - A - 0 011 067

CHEMICAL ABSTRACTS, vol. 91, no. 17, October 22, 1979, page 648, abstract 140734w, Columbus, Ohio, USA

**The file contains technical information submitted after the application was filed and not included in this specification**

(73) Proprietor: MERCK & CO. INC.
126, East Lincoln Avenue P.O. Box 2000
Rahway New Jersey 07065 (US)

(72) Inventor: Rokach, Joshua
416 Canterbury Place
Chomedey-Laval, Quebec (CA)
Inventor: Cragoe, Edward J.
2211 Oak Terrace Drive
Lansdale, Pennsylvania 19446 (US)
Inventor: Rooney, Clarence S.
2902 Hickory Hill Drive
Worcester, Pennsylvania 19490 (US)

(74) Representative: Crampton, Keith John Allen et al,
D YOUNG & CO 10 Staple Inn
London WC1V 7RD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 023 078 B1

Courier Press, Leamington Spa, England.

# Substituted dibenzo(b,f)thiepin-3-carboxylic acid derivatives, their preparation and pharmaceutical compositions containing them

This invention relates to prostaglandin antagonists, which are useful in treating a variety of conditions, such as allergic asthma, where excessive contractile activity of prostaglandins and prostaglandin biosynthetic intermediates occur.

Chemical Abstracts *91*, 648 (1979) 14073W, which refers to our published Japanese Patent Application JP—A—79 44691, which in turn corresponds to our European Patent Application EP—A—0 011 067, discloses *inter alia* prostaglandin antagonist compounds of the general formula:

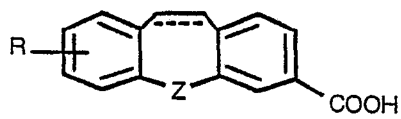

In this formula R is a hydrogen or halogen atom or certain organic or inorganic radicals, Z is thio, sulfinyl or sulfonyl, and the dotted line indicates either an olefinic bond or saturation at the 10(11) position, and their pharmaceutically acceptable salts.

The prostaglandin antagonists of the present invention are 8-fluorodibenzo[b,f]thiepines having the structural formula:

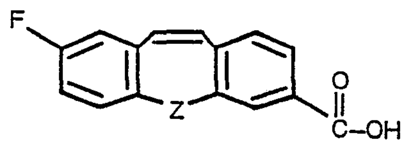

where Z is thio, sulfinyl or sulfonyl; or are pharmaceutically acceptable salts thereof; or 8-hydroxydibenzo[b,f]thiepin-3-carboxylic acid-5-oxides having the structural formula:

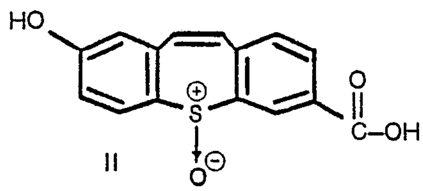

II

and the pharmaceutically acceptable salts thereof.

These dibenzo[b,f]thiepine derivatives antagonise the actions of contractile prostaglandins, such as $PFG_{2\alpha}$, $PGG_2$ and $PGH_2$ and $TXA_2$. The use of agents which act as prostaglandin antagonists offers new approaches to therapy in a number of disease states. For example, certain prostaglandins, such as $PGF_{2\alpha}$, $PGG_2$ and $PGH_2$, are potent contractants of bronchial muscle. Indeed human asthmatics have been shown to be especially sensitive to the bronchial constricting action of $PGF_{2\alpha}$.

In addition to the involvement of contractile prostaglandins in chronic obstructive lung disease (or asthma), prostaglandins are known to play a role in other allergic conditions, as well as inflammation, diarrhea, hypertension, angina, platelet aggregation, cerebral spasm, premature abortion, and dismenorrhea.

In addition to the prostaglandin antagonist actions, the dibenzo[b,f]thiepines of this invention are antagonists of slow reacting substance of anaphylaxis (SRS—A). This contractile substance is released in the lung tissues in allergic asthma, and antagonism of its actions contributes to alleviation of this disease.

The 8-fluorodibenzo[b,f]thiepines of formula I wherein Z is thio may be prepared according to the following general reaction scheme:

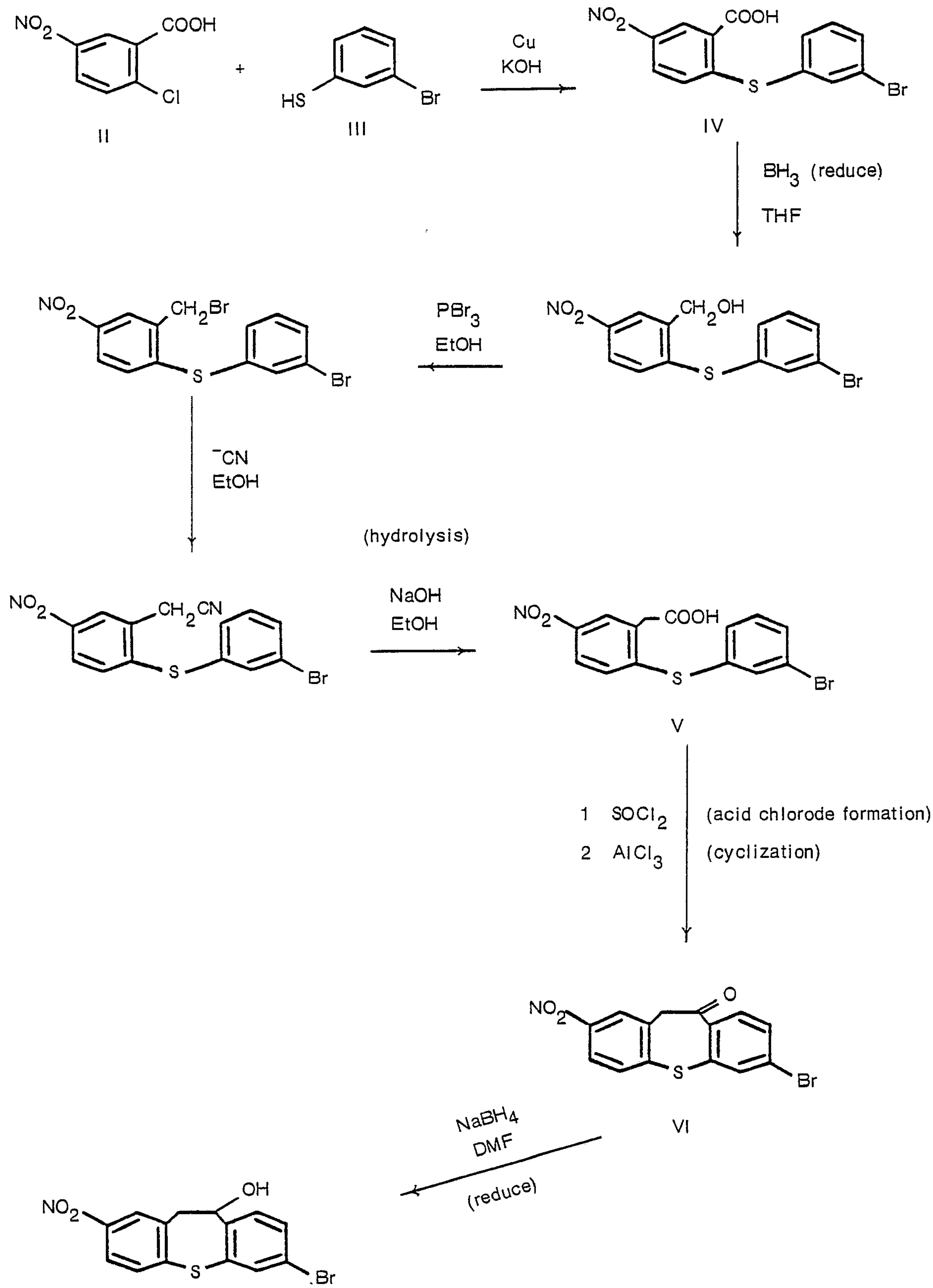
+
Cu
KOH
$BH_3$ (reduce)
THF
$PBr_3$
EtOH
$^-CN$
EtOH
(hydrolysis)
NaOH
EtOH
1 $SOCl_2$ (acid chlorode formation)
2 $AlCl_3$ (cyclization)
$NaBH_4$
DMF
(reduce)

II

III

IV

V

VI

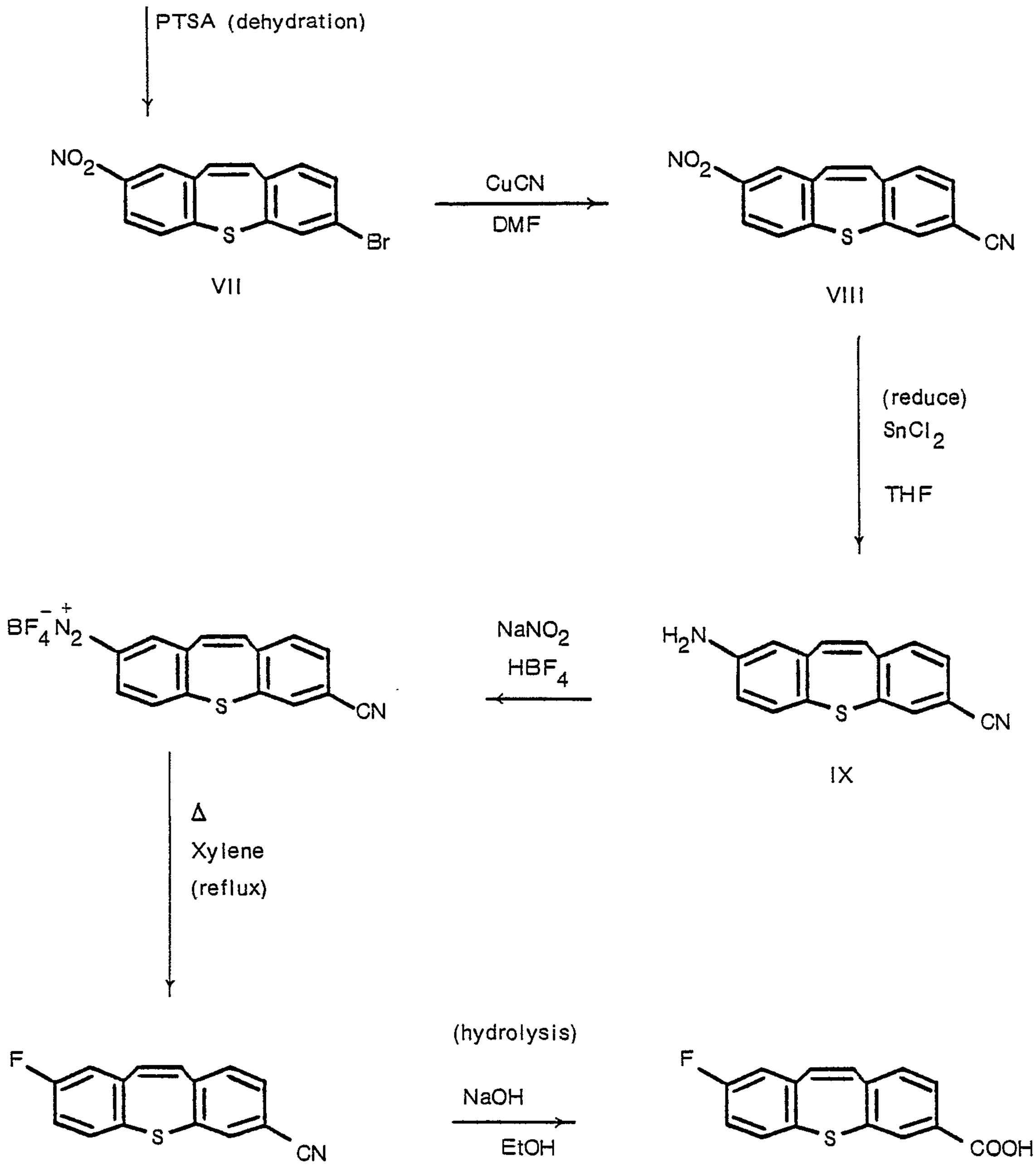
PTSA (dehydration)
VII
CuCN
DMF
VIII
(reduce)
$SnCl_2$
THF
$NaNO_2$
$HBF_4$
IX
Δ
Xylene
(reflux)
(hydrolysis)
NaOH
EtOH
X
XI

The 2-(3′-bromophenylthio)-5-nitrophenylacetic acid intermediates (V) also may be prepared from XII as shown in the following structural formulas:

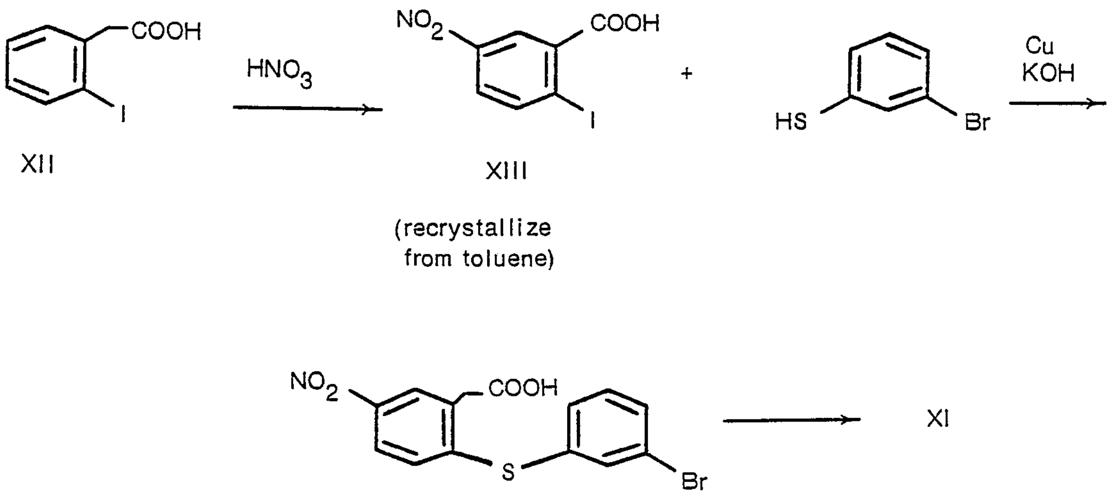


XII

XIII

(recrystallize from toluene)

V

XI

When 2-chlorophenylacetic acid is nitrated instead of 2-iodophenylacetic acid, the nitration yields predominantly the desired 5-nitro-2-chlorophenylacetic acid free of other nitro isomers. This compound also may be reacted with 3-bromothiophenol to form the intermediate 2-(3′-bromophenylthio)-5-nitrophenylacetic acid (V).

Alternatively, the 8-fluorodibenzo[b,f]thiepines of formula I wherein Z is thio may be prepared according to the following general reaction scheme:

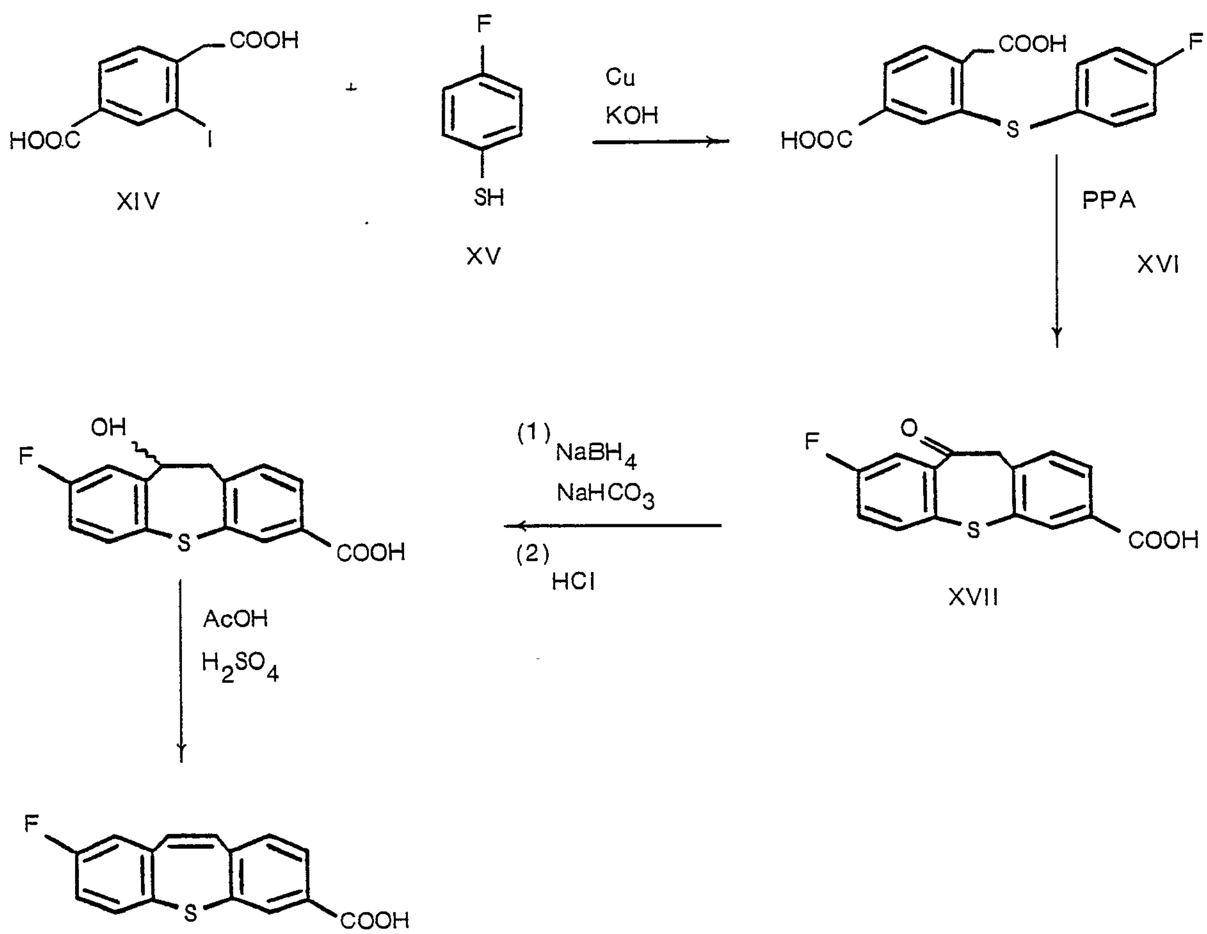


XIV

XV

XVI

XVII

XI

These compounds of formula I wherein Z is sulfinyl or sulfonyl may be prepared from the cyano intermediate (X) by controlled oxidation techniques. Thus, for example, 3-cyano-8-fluorodibenzo[b,f]thiepine (X) may be oxidized with hdyrogen peroxide in the presence of an acidic solvent, such as acetic acid, or with oranic peroxides, such as peroxy acids, for example m-chlorobenzoic acid (MCPBA) and the like, in a step-wise fashion to form the corresponding sulfinyl derivative (XVIII) and sulfonyl derivative (XIX) by controlling the molar ratio of oxidant to reductant. The molar ratio determines the oxidation level of the sulfur in the final product. A 1:1 molar ratio, for example, results largely in the production of the sulfinyl derivative whereas a 1 to 3 molar excess of oxidant results in a yield predominantly comprising the sulfonyl derivative.

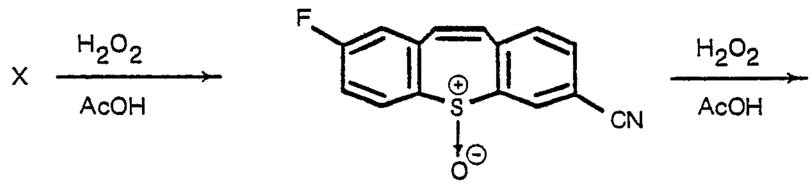


XVIII

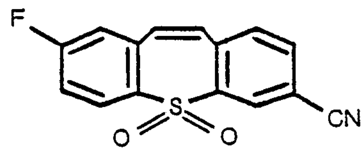

XIX

Hydrolysis of nitrile intermediates (XVIII) and (XIX) using aqueous molar mineral acid or alkali provides the corresponding carboxylic acids (XX) and (XXI).

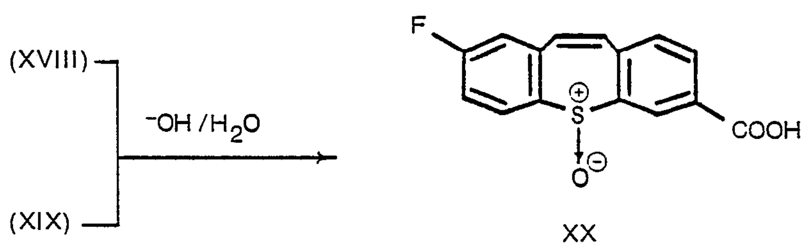


XX

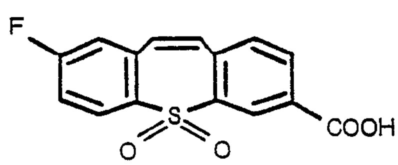

XXI

Alternatively, the sulfinyl and sulfonyl compounds of formula I may be prepared directly from the 8-fluoro-3-carboxylic acid (XI) employing the controlled oxidation techniques described above. If desired, although it is not essential, the 8-fluoro-3-carboxylic acid may be converted to a lower alkyl ester by reaction, for example, with a lower alkanol in the presence of a mineral acid, such as sulfuric acid, prior to oxidation. The sulfinyl (XXII) and sulfonyl (XXIII) esters so produced then are readily hydrolyzed to the desired carboxylic acid (XXIV) and (XXV).

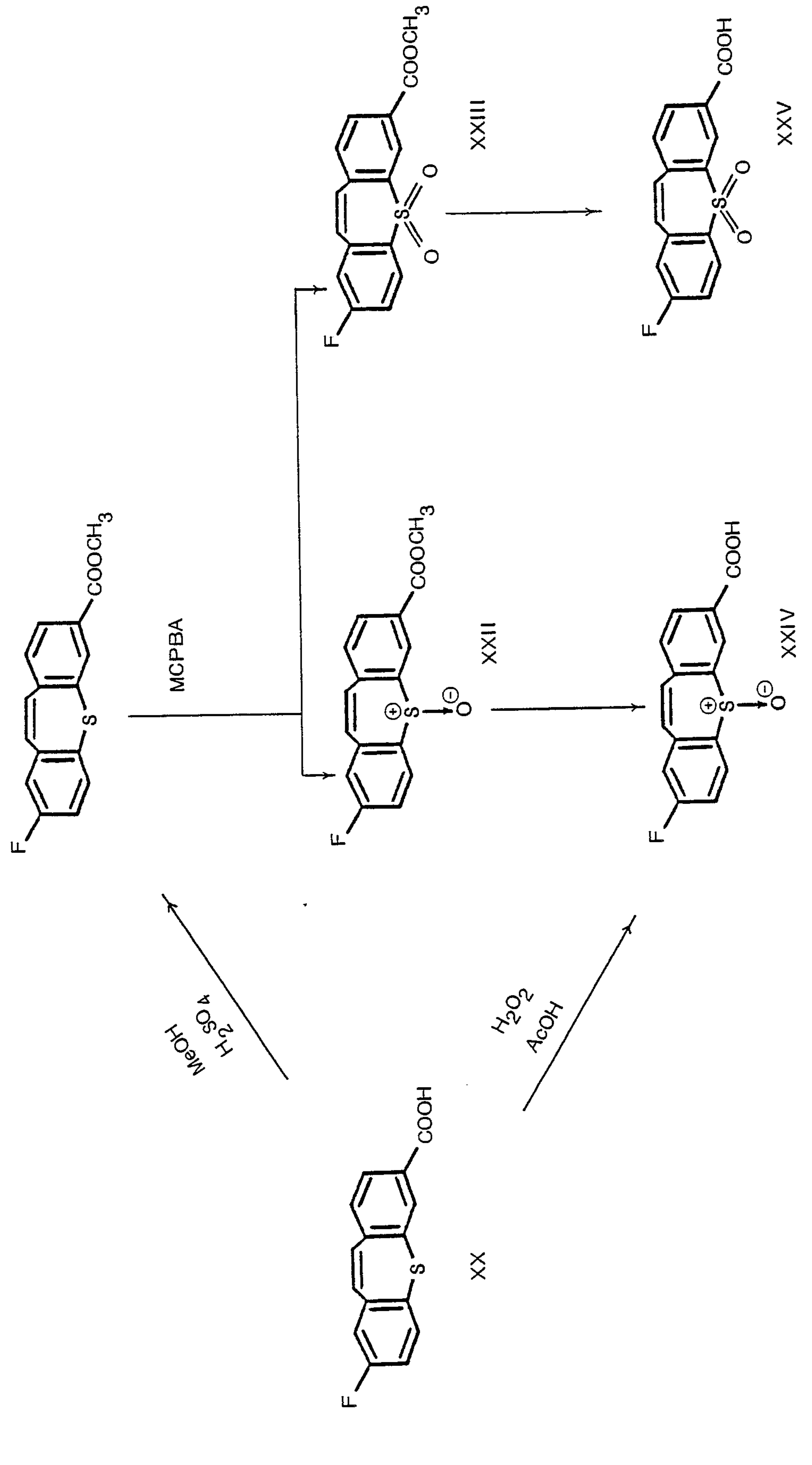

MeOH
$H_2SO_4$
$H_2O_2$
AcOH
MCPBA
XX
XXII
XXIII
XXIV
XXV

The 8-hydroxydibenzo[b,f]thiepine-5-oxide of formula II may be prepared according to the following general reaction scheme:

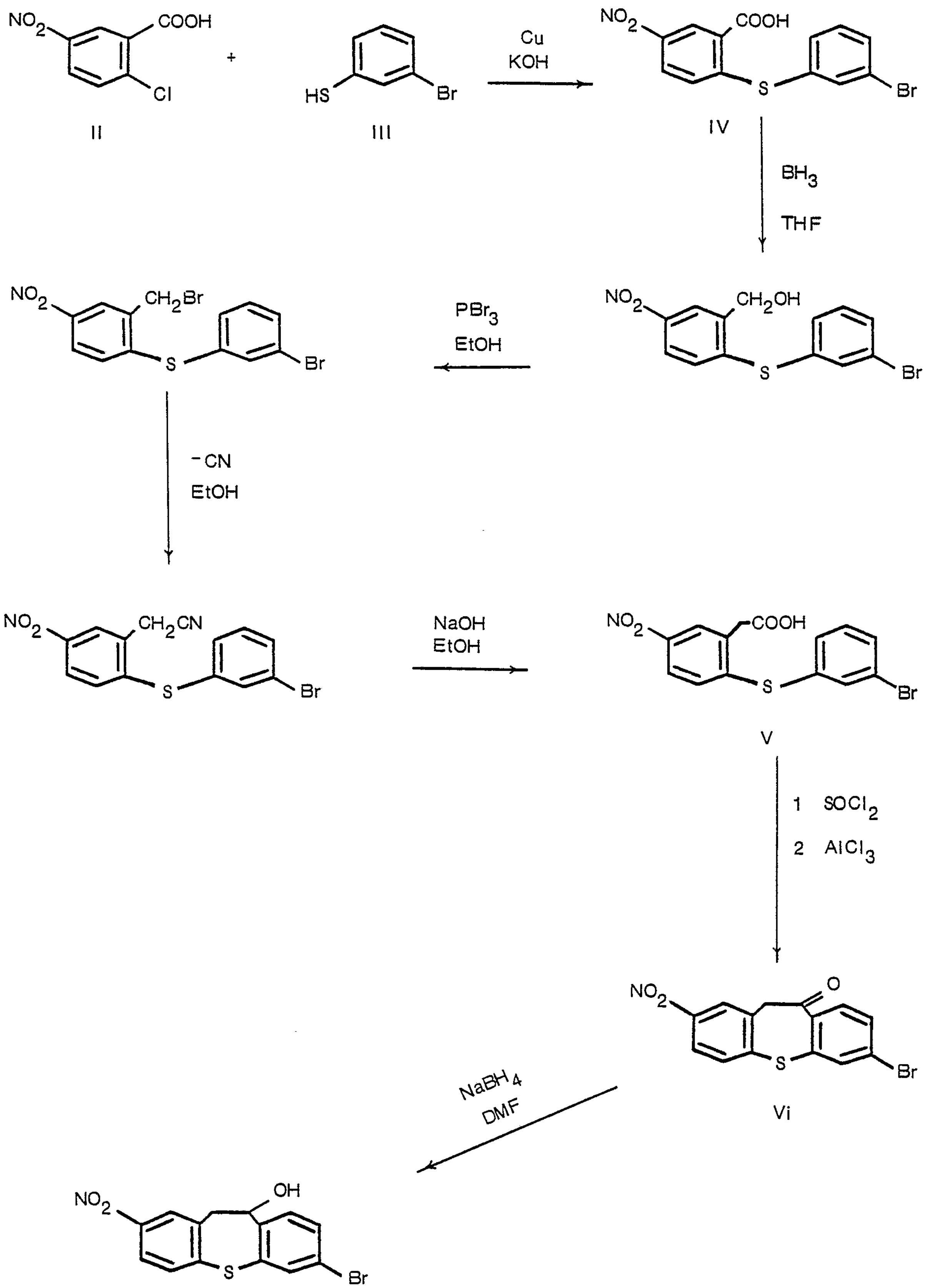

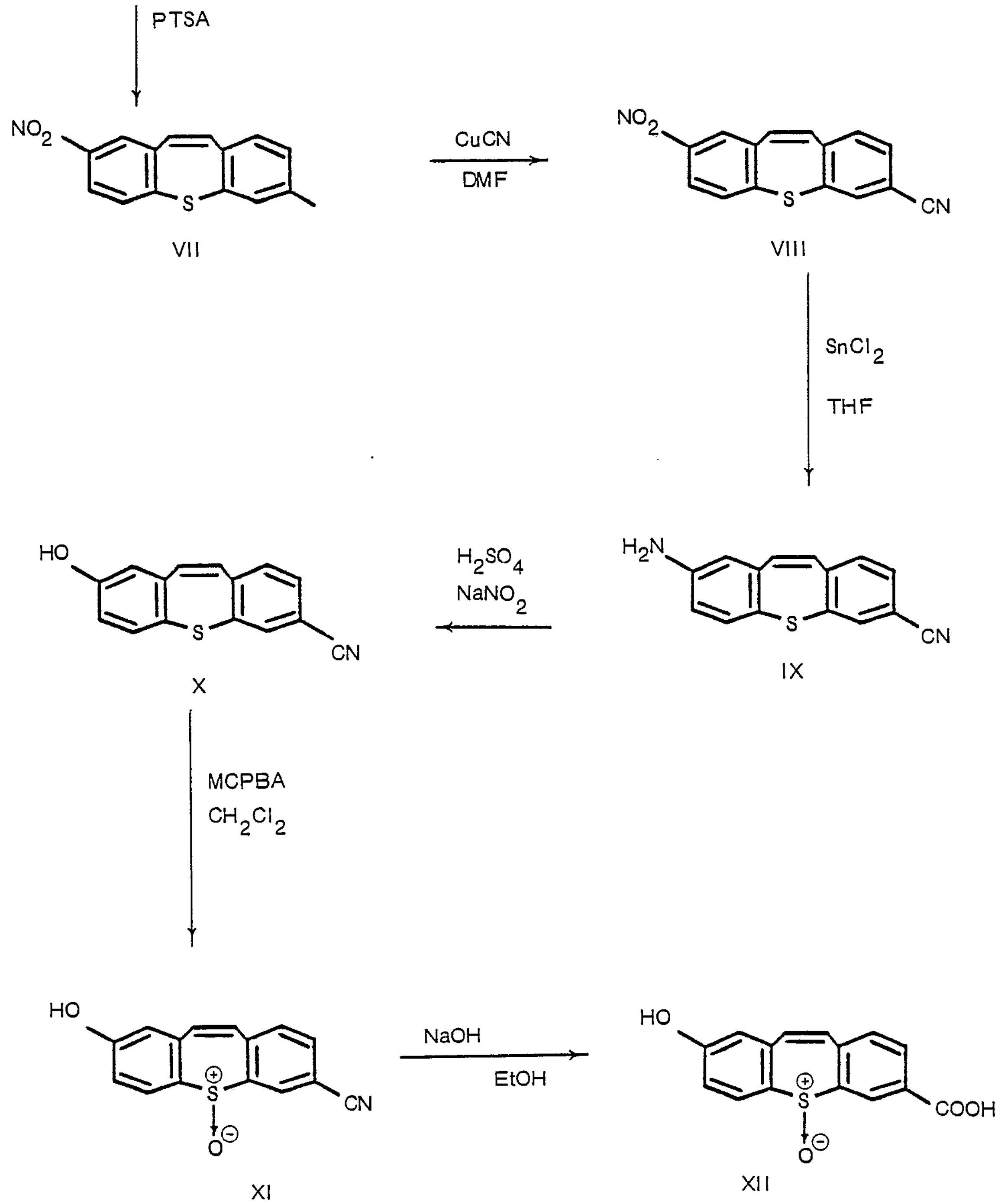


VII VIII IX X XI XII

The 2-(3′-bromophenylthio)-5-nitrophenyl-acetic acid intermediate (V) also may be prepared from 2-iodophenylacetic acid (XII) by the reactions illustrated below:

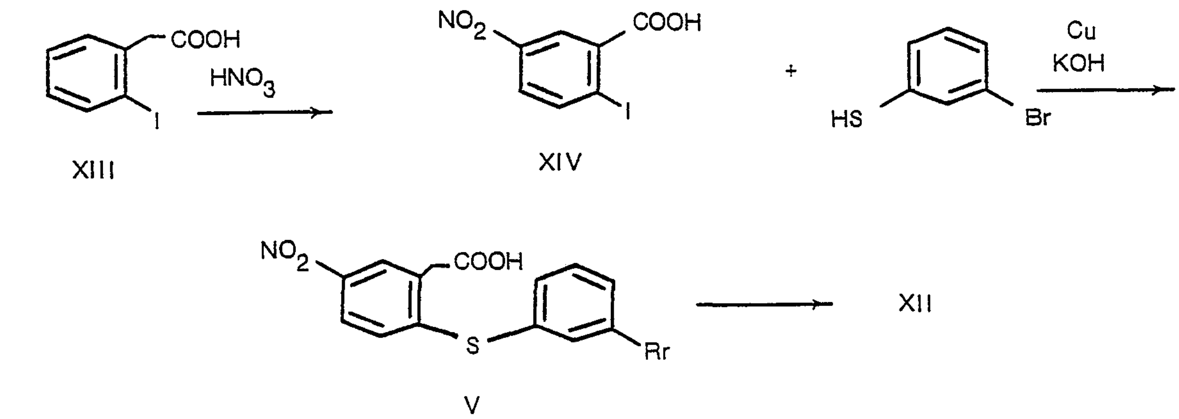


XIII XIV V XII

Alternatively, 8-hydroxydibenzo[b,f]thiepin-3-carboxylic acid may be prepared according to the following general reaction scheme:

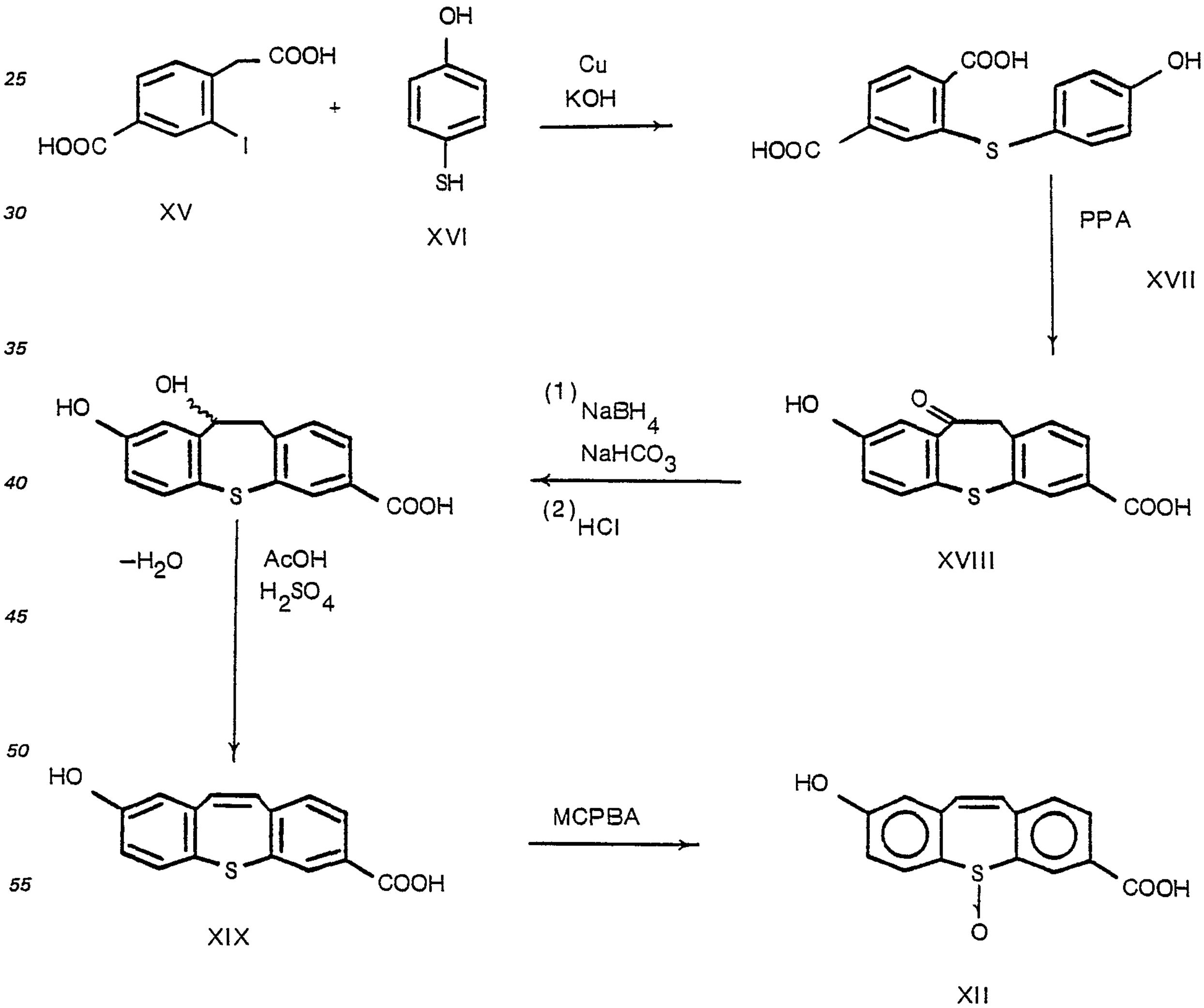


XV XVI XVII XVIII XIX XII

As noted above, the 5-sulfinyl (5-oxide) compounds of formula I may be prepared from the cyano intermediate (X) by controlled oxidation techniques. Thus, for example, 3-cyano-8-hydroxydibenzo[b,f]thiepine (X) may be oxidized with hydrogen peroxide in the presence of an acidic solvent, such as acetic acid, or with organic peroxides, such as peroxy acids, for example m-chloroperbenzoic acid (MCPBA) and the like, in a step-wise fashion to form the corresponding sulfiny

derivative (XI) and sulfonyl derivative (XX) by controlling the molar ratio of oxidant to reductant. The molar ratio determines the oxidation level of the sulfur in the final product. A 1:1 molar ratio, for example, results largely in the production of the sulfinyl derivative.

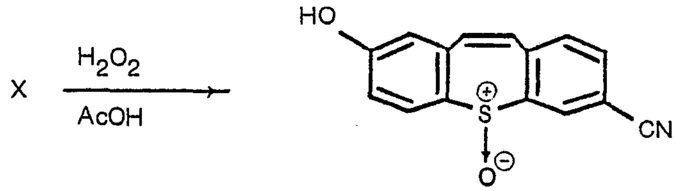


XI

Hydrolysis of nitrile intermediate (XI) using aqueous molar mineral acid or alkali provides the corresponding carboxylic acid (XII):

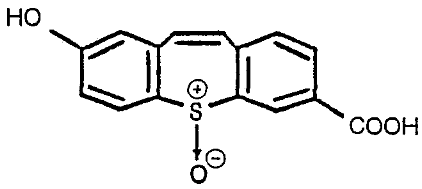

XII

Alternatively, these sulfinyl compounds may be prepared directly from the 8-hydroxy-3-carboxylic acid (XIX) employing the controlled oxidation techniques described above. If desired, although it is not essential, the 8-hydroxy-3-carboxylic acid may be converted to a lower alkyl ester by reaction, for example, with a lower alkanol in the presence of a mineral acid, such as sulfuric acid, prior to oxidation. The sulfinyl (XXII) esters so produced are readily hydrolyzed to the desired carboxylic acid (XII).

As noted above, pharmaceutically acceptable salts of the novel thiepines also are included within the scope of this invention. The term, pharmaceutically acceptable salts, is intended to include salts derived from pharmaceutically acceptable non-toxic bases such as, for example, ammonium salts, alkali metal salts such as sodium and potassium salts, alkaline earth metal salts such as magnesium and calcium salts, salts of organic bases such as amine salts derived from mono-, di and tri-loweralkyl or loweralkanol amines such as trimethylamine, dimethylamine and triethanolamine and salts derived from heterocyclic amines such as piperidine, pyrrolidine, piperazine and morpholine.

Example 1

8-Fluorodibenzo[b,f]thiepin-3-carboxylic Acid

Step A: *2-(3'-Bromophenylthio)-5-nitrobenzoic Acid*

Dissolve 188 g of potassium hydroxide pellets in 2 liters of waer and add 205 g of 3-bromothiophenol. Stir the mixture for 10 minutes and add 6.5 g of copper metal powder followed by 205 g of 2-chlorol-5-nitrobenzoic acid. Reflux the mixture for 90 minues and filter while hot. Dilute the filtrate with water and heat to re-dissolve solids, and acidify with concentrated hydrochloric acid. Separate the solids by filtration, wash with water and dry. Stir the residue in 3 liters of ether for 5 hours, filter and dry the residue to obtain the title product. (mp 202—206°C).

Step B: *2-(3'-Bromophenylthio)-5-nitrobenzyl Alcohol*

Dissolve 285.3 g of the nitrobenzoic acid of Step A in 1 liter of tetrahydrofuran. Stir under a nitrogen atmosphere at room temperature and add slowly 1250 ml of a 1M solution of borane in tetrahydrofuran. Continue stirring for 2 hours. Slowly add 300 ml of water with exterior cooling. Partition the resulting mixture with between water and ethyl acetate. Separate the organic layer, wash with water, dry, charcoal, and strip to a solid residue. (mp 98—100°C).

Step C: *2-(3'-Bromophenylthio)-5-nitrobenzyl Bromide*

To 225 g of the alcohol of Step B, add dropwise 27 ml of phosphorous tribromide. Stir the mixture for 10 minutes then allow to stand for 30 minutes. Dilute with water and stir the mixture for 30 minutes. Dilute with ethyl acetate to dissolve all solids in the organic phase. Separate the organic phase, extract the aqueous phase twice with ethyl acetate and combine the organic phases. Wash the combined ethyl acetate extracts with water and dry over sodium sulfate. Filter and evaporate the filtrate to dryness to obtain the title product. (mp 80—82°C).

Step D: *2-(3'-Bromophenylthio)-5-nitrobenzyl Cyanide*

Wash well with water 500 g of Amberlite IRA—400 resin (chloride form), Rohm & Haas Co.,

Philadelphia, Pennsylvania. Stir the washed resin for 10 minutes with a solution of 60 g of sodium cyanide in 300 ml of water. Dilute with 200 ml of water and stir for 10 minutes. Allow the resin to settle and decant the supernatant. Repeat treatment 3 times. Wash the treated resin to neutrality with water and air dry.

Suspend 85 g of benzyl bromide of Step C in 2 liters of absolute ethanol and add 100 gm of the treated resin prepared above. Stir the mixture under reflux for 2 hours. Add 100 g of resin and continue stirring for 1 hour. Filter while hot and wash the resin with tetrahydrofuran. Strip the filtrate to dryness to obtain the title product as a 2:1 mixture of dialkylated nitrile product (70 g). Employ in the next step without further purification.

Step E: *2-(3'-Bromophenylthio)-5-nitrophenylacetic Acid*

Suspend the material obtained in Step D (70 gm) in 700 ml of acetic acid and add 600 ml of concentrated hydrochloric acid. Reflux the mixture for 8 hours. Cool the mixture and decant the supernatant. Evaporate the supernatant to dryness to obtain a first crop.

Suspend the residue remaining after decantation in 700 ml of acetic acid and 600 ml of concentrated hydrochloric acid and reflux for 8 hours. Decant the supernatant and evaporate to dryness to obtain a second crop.

Suspend the combined crops in 1 liter of ethyl acetate. Filter to remove any insolubles and extract twice with 200 ml of 5% aqueous sodium hydroxide. Combine the aqueous layers and acidify with 20% aqueous hydrochloric acid. Extract twice with 500 ml of ethyl acetate. Wash the ethyl acetate layer with water, dry over sodium sulfate, filter and evaporate to dryness to obtain the title product (m.p. 123—125°C).

Step F: *3-Bromo-8-nitro-10,11-dihydro-11-oxodibenzo[b,f]thiepine*

Add to 45 g of the acid of Step E, 40 ml of thionyl chloride and stir the mixture at reflux for 15 minutes. Evaporate the excess thienyl chloride. Dissolve the residue in benzene and evaporate to dryness. Again dissolve the residue in benzene and evaporate to dryness. To the residue, add 1300 ml of 1,2-dichloroethane. Stir the mixture and add in portions 17.5 g of aluminum chloride. Continue stirring at room temperature for 30 minutes. Pour the mixture into 1 liter of water. Extract twice with 700 ml of 1,2-dichloroethane. Wash the organic phase with water, dry over sodium sulfate, filter and evaporate to dryness to obtain the title product. (m.p. 242—245°C).

Step G: *3-Bromo-8-nitro-11-hydroxy-10,11-dihydrodibenzo[b,f]thiepine*

Suspend 39 g of the 11-keto compound of Step F in 600 ml of tetrahydrofuran and add 4 g of sodium borohydride. Stir at room temperature for 12 hours. Dilute the reaction mixture with water and extract twice with 500 ml of ether. Combine the organic phases, dry over sodium sulfate, filter and evaporate to dryness to obtain the title product which is used as such in the next step (yield 38.6 g crude).

Step H: *3-Bromo-8-nitrodibenzo[b,f]thiepine*

Suspend 72 g of the 11-hydroxy compound of Step G in 3 liters of toluene and bring the mixture to reflux using a Dean-Stark trap. Continue refluxing for 2 hours. Add 7.5 g of p-toluenesulfonic acid and continue refluxing for 1 hour. Cool the reaction mixture, wash twice with 200 ml of 10% aqueous sodium carbonate solution, and wash to neutrality with water. Dry over sodium sulfate, filter and evaporate to dryness to obtain the title product. (m.p. 190—192°C).

Step I: *3-Cyano-8-nitrodibenzo[b,f]thiepine*

Dissolve 21 g of the bromo-nitro compound of Step H in 250 ml of dimethylformamide and add 17 g of cuprous cyanide. Reflux the mixture for 12 hours, cool and acidify with 6N hydrochloric acid. Stir for 15 minutes at room temperature, dilute with water and separate the solids by filtration. Take up the residue in ethyl acetate, wash with water, dry over sodium sulfate, filter and evaporate the filtrate to dryness to obtain the title product. (m.p. 234—236°C).

Step J: *3-Cyano-8-aminodibenzo[b,f]thiepine*

Dissolve 5.6 g of the cyano-nitro compound of step I in 600 ml of tetrahydrofuran and add 27 g of stannous chloride in 40 ml of water and 100 ml of concentrate hydrochloric acid. Stir the mixture at room temperature for 24 hours, pour over ice and shake the resulting mixture with 300 ml of 20% aqueous sodium hydroxide. Separate the organic layer and shake the aqueous basic layer with tetrahydrofuran. Separate the organic layer. Combine the organic extracts, dry and evaporate to dryness. Chromatograph the residue on silica gel eluting with 20% ethyl acetate in benzene to obtain the title product. (m.p. 181—183°C).

Step K: *3-Cyano-8-fluorodibenzo[b,f]thiepine*

Suspend 6 g of 3-cyano-8-aminodibenzo[b,f]thiepine in 15 ml of 50% fluoboric acid ($HBF_4$) and 15 ml of water. Cool the mixture to 3—5°C. and slowly add 18 ml of of 10% sodium nitrate. Stir in the

cold for 4 hours and add urea to destroy the excess sodium nitrite. Separate the solids by filtration and wash with cold 5% fluoboric acid followed by cold water and then ether. Air dry. Suspend the diazonium fluoroborate in 300 ml of xylene and reflux for 2 hours with stirring. Filter and evaporate the filtrate to dryness. Triturate with ether to obtain the title product. (m.p. 146—148°C).

Step L: *8-Fluorodibenzo[b,f]thiepin-3-carboxylic Acid*

Suspend 300 mg of the cyano compound of Step A in 8 ml of 20% aqueous sodium hydroxide and 8 ml of absolute ethanol. Reflux under nitrogen for 2 hours. Evaporate the ethanol. Acidify the suspension with 20% hydrochloric acid and separate the solids by filtration. Dissolve the residue in 20 ml of hot methanol and add a few drops of concentrated hydrochloric acid. Evaporate the methanol, dilute with ether and evaporate again. Dilute with water, stir for 30 minutes and filter. Wash the solids with water and dry *in vacuo* at 100°c to obtain the title product. (m.p. 270—272°C).

Example 2

8-Fluorodibenzo[b,f]thiepin-3-carboxylic Acid

Step A: *2-(4'-fluorophenylthio)-4-carboxyphenyl)-acetic Acid*

Dissolve 35 g of potassium hydroxide pellets in 375 ml of water. Add 38.3 g of 2-iodo-4-carboxyphenylacetic acid followed by 22.2 g of 4-fluorothiophenol and 11.9 g of copper powder. Stir under a nitrogen atmosphere and heat to 105—110°C for 56 hours. Filter while hot and acidify the filtrate with 20% hydrochloric acid. Separate the solids by filtration, wash with water and dry *in vacuo* at 60°C (36.9 g — 96.3% crude). Dried solids may be used as such in the next step. Purify by stirring at room temperature in ethyl acetate for 6 hours. Filter to obtain title product. (m.p. 218—221°C).

Step B: *8-Fluoro-10-oxo-10,11-dihydrodibenzo[b,f]thiepin-3-carboxylic Acid*

Heat to 120°C with stirring 450 g of commercial polyphosphoric acid and add 3.59 g of crude diacid from Step A. Stir under a nitrogen atmosphere at 120°C for 90 minutes. Cool and slowly add 1.5 liters of ice and water. Separate the solids by filtration. Stir the residue in 500 ml of refluxing ethyl acetate for 90 minutes and filter while hot to obtain a first crop. Concentrate the filtrate until solids appear, allow to stand overnight and filter to obtain a second crop. Combine the crops (18.26 g — 54% crude) which may be used in the next step as such. Purify by boiling in ethyl acetate for 15 minutes, hold at room temperature overnight and filter. (m.p. 289—291°C).

Step C: *8-Fluoro-10-hydroxy-10,11-dihydrodibenzo[b,f]thiepin-3-carboxylic Acid*

Suspend 15.5 g of the crude 10-keto compound of Step B in 750 ml of water and add 10 g of sodium bicarbonate. Stir the mixture and warm to 50°C for 15—20 minutes. Stop heating and slowly add 5 g of sodium borohydride. Acidify the mixture with 10% aqueous hydrochloric acid, separate the solids by filtration, wash with water and air-dry. The residue may be used as such in the next step. (m.p. 204—210°C, subsequent gassing).

Step D: *8-Fluorodibenzo[b,f]thiepin-3-carboxylic Acid*

Suspend 14.8 g of the crude alcohol from Step C in 300 ml of glacial acetic acid and heat the mixture with stirring in an oil-bath at 110—115°C. Add 50 ml of sulfuric acid in a thin stream. Continue stirring and heating for 15 minutes. Cool and pour into 500 ml of water. Separate the solids by filtration and stir at room temperature in 125 ml of ethylacetate overnight. Separate the solids by filtration to obtain the title product. (m.p. 273—275°C).

Example 3

8-Fluorodibenzo[b,f]thiepin-3-carboxylic Acid-5-oxide and 8-Hydroxydibenzo[b,f]thiepin-3-carboxylic Acid-5-oxide

Step A: *3-Cyano-8-fluorodibenzo[b,f]thiepin-5-Oxide*

Dissolve 1.7 g of 3-cyano-8-fluorodibenzo[b,f]thiepine in 90 ml. of methylene chloride and add 1.36 g of 85% m-chloroperbenzoic acid. Stir the mixture at room temperature for 90 minutes. Add 4 g of calcium hydroxide over 15 minutes. Filter and strip the filtrate to dryness. Triturate the residue in 30 ml. of ether for 2 hours. Separate the solids by filtration to obtain the title product. (mp. 240—243°C.).

Step B: *8-Fluorodibenzo[b,f]thiepin-3-carboxylic Acid-5-Oxide*

Suspend 1.5 g of the cyano compound of Step A in 50 ml. of acetic acid and add 50 ml. of 50% sulfuric acid. Reflux for 10 hours. Filter and dilute the filtrate with water. Separate the solids by filtration and dissolve in hot acetic acid. Charcoal the hot solution, filter and allow to crystallize to obtain the title product (mp. 267—269°C).

Step C: *3-Cyano-8-hydroxydibenzo[b,f]thiepine*

Suspend 50 mg. of 3-cyano-8-aminodibenzo[b,f]thiepine in 2 ml. of 10% sulfuric acid and cool to 3°C. Add dropwise 0.2 ml. of 10% aqueous sodium nitrite solution. Stir at 3°C for 2 hours and allow to warm to 15°C for 1 hour. Add the solution dropwise to 50 ml. of boiling 10% sulfuric acid and continue

heating for 15 minutes. Dilute with cold water and filter. Wash the solids with water and dry to obtain the title product. (mp. 224—227).

Step D: *3-Cyano-8-hydroxydibenzo[b,f]thiepin-5-Oxide*

Suspend 50.2 mg. of 3-cyano-8-hydroxydibenzo[b,f]thiepine in 9 ml. of methylene chloride and add 40.6 mg. of m-chloroperbenzoic acid. Allow the mixture to stand at room temperature for 30 minutes. Separate the solids by filtration to obtain the title product. (mp. 241—243°C.).

Step E: *8-Hydroxydibenzo[b,f]thiepin-3-carboxylic Acid-5-Oxide*

Suspend 100 mg. of the cyano compound of Step D in 5 ml. of acetic acid and 5 ml. of 20% aqueous sodium hydroxide. Reflux under nitrogen for 1 hour. Evaporate the alcohol and dilute the residue with water. Extract with ether and acidify the aqueous phase with 6 N hydrochloric acid. Separate the solids by filtration. Dissolve the solids in ethanol and strip to dryness to obtain the title product. (mp. 288—291°C. dec.).

Example 4

8-Fluorodibenzo[b,f]thiepin-3-carboxylic Acid-5-5-Dioxide

Suspend 1.3 g of 8-fluorodibenzo[b,f]thiepin-3-carboxylic acid in 35 ml of glacial acetic acid. Heat the mixture in an oil-bath at 100°C and add 3.5 ml of 30% hydrogen peroxide. Continue heating for 105 minutes. Dilute the mixture with 200 ml of water and separate the solids by filtration. Stir the residue overnight at room temperature in 15 ml of ethyl acetate. Separate the solids by filtration to obtain the title product. (m.p. 244—248°C).

8-Fluorodibenzo[b,f]thiepin-3-carboxylic acid-5-oxide can be resolved into a highly potent antiasthmatic agent, viz. 8-fluorodibenzo[b,f]thiepin-3-carboxylic acid-5-oxide R(—) isomer, by converting it to a mixture of diastereoisomers by reaction with an optically active reagent. The mixture of diastereoisomers is then separated into the component isomers, e.g. by chromatography or crystallization, and the desired enantiomer is regenerated from the appropriate diastereoisomer.

For example, an optically active ester of the acid to be resolved can be prepared by first converting the acid to an acid halide or mixed anhydride, followed by reaction with an optically active alcohol such as (+)-2-phenyl propanol, followed by oxidation to the corresponding 5-oxide, to form the diastereoisomeric pair of esters and separation of the esters, e.g. by chromatography or crystallization. The desired acid is regenerated from the appropriate ester by acid or basic hydrolysis.

Preferably, the 8-fluorodibenzo[b,f]thiepin-3-carboxylic acid or its corresponding 5-oxide is converted to the amide of an optically active amine such as *L*(—)-*α*-methylbenzylamine, and then oxidized with an organic per acid if the non-oxidized acid is the starting product, thus producing a mixture of diastereoisomer S(+)-8-fluorodibenzo[b,f]thiepin-3-(—)-N-*α*-methylbenzylcarboxamide-5-oxide and diastereoisomer R(—)-8-fluorodibenzo[b,f]thiepin-3-(—)-N-*α*-methylbenzylcarboxamide-5-oxide. This mixture is then separated by column chromatography using silica gel as the support medium and 15 percent v/v ethyl acetate in chloroform as the developing solvent. Following elution from the column, the desired diastereoisomer R(—)-8-fluorodibenzo[b,f]thiepin-3-(—)(N-*α*-methylbenzyl)carboxamide-5-oxide is recovered from the more polar fractions which are eluted primarily from the later fractions collected from the column.

The separated diastereoisomeric amide is then hydrolysed, preferably under acid conditions, and the desired enantiomer 8-fluorodibenzo[b,f]thiepin-3-carboxylic acid-5-oxide R(—) isomer recovered as a crystalline solid.

Example 5

Preparation of 8-fluorodibenzo[b,f]thiepin-3-N-*α*-methylbenzylcarboxamide

9.52 g of 8-fluorodibenzo[b,f]thiepin-3-carboxylic acid (35 mmoles) is dissolved in 350 cc tetrahydrofuran, and 4.24 g triethylamine (42 mmoles) is added. The solution is cooled in an ice-water bath, and a solution of 4.18 g ethyl chloroformate (38.5 mmoles) in 10 cc tetrahydrofuran is added dropwise. This suspension is stirred in the cold for 15 minutes, then a solution of 8.49 g L-(—)-*α*-methylbenzylamine (70 mmoles) in 15 cc tetrahydrofuran is added dropwise. The resulting mixture is stirred in the cold for one hour and then filtered. The filtrate is stripped down. The residue is dissolved in chloroform, concentrated, and injected on a column of silica gel, and elution is carried out with 5 volume percent ethylacetate/toluene. There is obtained 0.65 g pure title product as an oil which is crystallized from toluene-hexane, m.p. 128—130°C.

Example 6

Preparation of 8-fluorodibenzo[b,f]thiepin-3-N-*α*-methylbenzylcarboxamide-5-oxide as a mixture of DL and LL diastereoisomers

7.05 g of 8-fluorodibenzo[b,f]thiepin-3-N-*α*-methylbenzylcarboxamide is dissolved in 350 cc methylene chloride, and the solution cooled in an ice-water bath. 3.65 g of 85 percent *m*-chloroperbenzoic acid (95 percent of 19 mmoles) is added, the mixture is stirred in the cold for one hour and then diluted with 125 cc methylene chloride, and 6 g of calcium hydroxide is added. The

mixture is stirred for 5 minutes, then filtered through Celite. The filtrate is stripped to an oil which is chromatographed to yield the title compound on 400 g silica gel.

Example 7

Separation of diastereoisomers

The mixture of diastereoisomers is chromatographed using column chromatography on a column packed with silica gel in 15% (v/v) ethyl acetate-chloroform. The enriched fraction of the least polar diastereoisomer A and the more polar diastereoisomer B are crystallized from toluene. The least polar fraction is eluted primarily in the early fractions and the more polar fraction is eluted primarily in the later fractions. Diastereoisomer S(+)-8-fluorodibenzo[b,f]thiepin-3-(—)-N-$\alpha$-methylbenzylcarboxamide-5-oxide (diastereoisomer A) has a m.p. of 224—226°C. Diastereoisomer R(—)-8-fluorodibenzo[b,f]thiepin-3-(—)-N-$\alpha$-methylbenzylcarboxamide-5-oxide (diastereoisomer B) has a m.p. of 187—188°C.

Example 8

Hydrolysis of diastereoisomer S(+)-8-fluorodibenzo[b,f]thiepin-3-(—)-N-$\alpha$-methylbenzyl carboxamide-5-oxide to 8-fluorodibenzo[b,f]thiepin-3-carboxylic acid-5-oxide S(+)isomer

475 mg of diastereoisomer S(+)-8-fluorodibenzo[b,f]thiepin-3-(—)-N-$\alpha$-methylbenzyl carboxamide-5-oxide is heated in a mixture of 10 cc acetic acid and 10 cc 50% aqueous sulfuric acid at 100—105°C under a nitrogen atmosphere for 7 hours. Gradually the solid material dissolves, then a new solid separates. Reaction is followed by thin-layer chromatography, and, as seen before, the intermediate primary amide is detected by thin-layer chromatography. The mixture is stopped by a timer after 7 hours and allowed to stand overnight.

The mixture is diluted with 30 cc water and the crystalline white solid is filtered and washed well with water, allowed to dry, triturated in ether, and filtered. There is obtained 310 mg of white fluffy solid, m.p. 257—259°C.

The rotation of a 1 percent solution in aqueous sodium bicarbonate is +55.80°.

Example 9

Hydrolysis of R-(—)-8-fluorodibenzo[b,f]thiepin-3-(—)-N-$\alpha$-methylbenzylcarboxamide-5-oxide to 8-fluorodibenzo[b,f]thiepin-3-carboxylic acid-5-oxide R(—)isomer

1.55 g of R(—)-8-fluorodibenzo[b,f]thiepin-3-(—)-N-$\alpha$-methylbenzylcarboxamide-5-oxide is heated in a mixture of 50 cc glacial acetic acid and 50 cc sulfuric acid under nitrogen atmosphere and at 100—105°C for 12 hours. The mixture, which contains crystals of 8-fluorodibenzo[b,f]thiepin-3-carboxylic acid-5-oxide R(—) isomer, is diluted with 100 cc water, stirred for 5 minutes, and filtered, and the solid is sucked dry for one and one-half hours, then stirred in ether (50 cc) for 15 minutes, and filtered. There is obtained 930 mg of white fluffy solid, m.p. 255—257°C.

The rotation of a 1% solution in aqueous sodium bicarbonate is —67.63 degrees.

The compounds of formulas I and II are useful in the treatment or prophylaxis of mammalian disease conditions where excessive undesirable contractile activity of prostaglandins, such as $PGF_{2\alpha}$, or prostaglandin biosynthetic intermediates contribute. These conditions include asthma, inflammatory states such as arthritis and asthma, as well as allergy, diarrhea, hypertension, angina, platelet aggregation, cerebral spasm, premature abortion, and dysmenorrhea. In particular, they are of value in reaginic mediated asthma (extrinsic asthma).

The magnitude of a prophylactic or therapeutic dose of compound of formula I or II will, of course, vary with the nature and the severity of the condition to be treated and with the particular compound of formula I or II and its route of administration. In general, the daily dose range lies within the range of 0.2 mg. to 100 mg. per kg. body weight of a mammal.

For use where a composition for intravenous administration is employed, a suitable dosage range is from 0.2 to 10 mg. (preferably 1 to 5 mg.) of a compound of formulas I or II per kg. of body weight per day; and in the case where an oral composition is employed, a suitable dosage range is about, e.g., 1 to 50 mg. of a compound of formula I or II per kg. of body weight per day, preferably from 10 to 40 mg./kg.

Pharmaceutical compositions including compounds I or II of the present invention suitable for oral administration and by inhalation in the case of asthma therapy may be presented as discrete units such as capsules, cachets, or tablets, each containing a predetermined amount of the active ingredient; as a powder of granules; or as a solution or a suspension in an aqueous liquid, a non-aqueous liquid, an oil-in-water emulsion, or a water-in-oil liquid emulsion.

**Claims**

1. A compound of the formula:

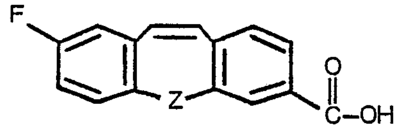

where Z is thio, sulfinyl or sulfonyl; or

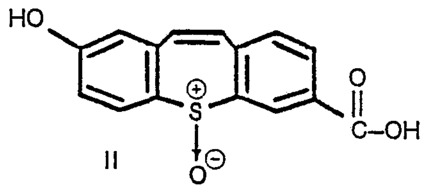

II

or a compound that is a pharmaceutically acceptable salt thereof.

2. 8-Fluorodibenzo[b,f]thiepin-3-carboxylic acid.

3. 8-Fluorodibenzo[b,f]thiepin-3-carboxylic acid-5-oxide.

4. The R(—) isomer of 8-fluorodibenzo[b,f]thiepin-3-carboxylic acid-5-oxide.

5. 8-Fluorodibenzo[b,f]thiepin-3-carboxylic acid-5,5-dioxide.

6. 8-Hydroxydibenzo[b,f]thiepin-3-carboxylic acid-5-oxide.

7. A composition for treating undesirable contractile activity of prostaglandins consisting essentially of a pharmaceutically acceptable carrier and a therapeutically effective amount of a compound as claimed in any of Claims 1 to 6.

8. A compound as claimed in any one of Claims 1 to 6 for use in treating undesirable contractile activity of prostaglandins in a host in the need of such treatment.

9. The compound claimed in Claim 4 for use in treating asthmatic diseases.

10. A process for preparing a compound of the formula:

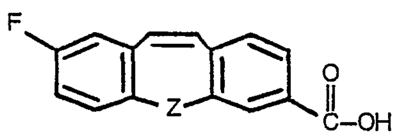

where Z is thiol sulfinyl or sulfonyl, or

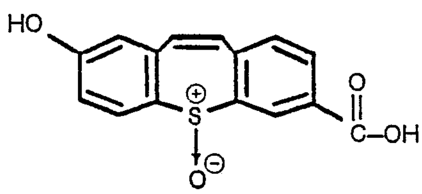

which comprises hydrolysing the corresponding compound having a 3-cyano substituent instead of a 3-carboxyl substituent with an acid or a base to produce the desired compound.

11. A method of preparing an optical isomer of 8-fluorodibenzo[b,f]thiepin-3-carboxylic acid-5-oxide present in the R(—) configuration, which has the formula:

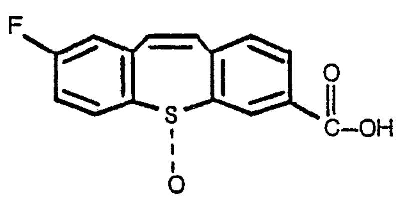

that comprises regenerating the carboxylic acid from a diastereoisomeric compound of the optically active acid and an optically active form of an ester or an amide.

12. A method according to claim 11 that comprises separating a mixture of diastereoisomers of an optically active amide of 8-fluorodibenzo[b,f]thiepin-3-carboxylic acid-5-oxide by chromatography

into a less polar fraction of a diastereoisomer of S(+)-8-fluorodibenzo[b,f]thiepin-3-(—)(N-α-methylbenzyl)carboxamide-5-oxide and a more polar fraction of a diastereoisomer of R(—)-8-fluorodibenzo[b,f]thiepin-3-(—)-(N-α-methylbenzyl)carboxamide-5-oxide, and hydrolysing the amide diastereoisomer R(—)-8-fluorodibenzo[b,f]thiepin-3-(—)-N-α-methylbenzylcarboxamide-5-oxide fractions separately to produce 8-fluorodibenzo[b,f]thiepin-3-carboxylic acid-5-oxide R(—) isomer.

13. A method according to claim 11 that comprises converting the said acid to the corresponding acid halide, contacting the acid halide with an optically active amine to form 8-fluorodibenzo[b,f]thiepin-3-N-substituted carboxamide-5-oxide as a mixture of DL and LL diastereoisomers, separating the diastereoisomeric mixture by chromatography into the less polar diastereoisomer S(—)-8-fluorodibenzo[b,f]thiepin-3-(—)-N-substituted carboxamide-5-oxide and the more polar diastereoisomer R(—)8-fluorodibenzo[b,f]thiepin-3-(—)N-substituted carboxamide-5-oxide, and subjecting the R(—)8-fluorodibenzo[b,f]thiepin-3-(—)-N-substituted carboxamide-5-oxide to acid hydrolysis to form 8-fluorodibenzo[b,f]thiepin-3-carboxylic acid-5-oxide R(—) isomer.

14. A method as claimed in claim 11, in which the amine is *L*-α-methylbenzylamine.

**Patentansprüche**

1. Eine Verbindung der Formel:

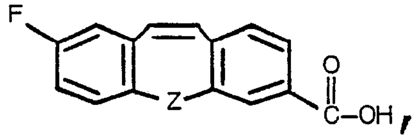

worin Z Thio, Sulfinyl oder Sulfonyl ist; oder

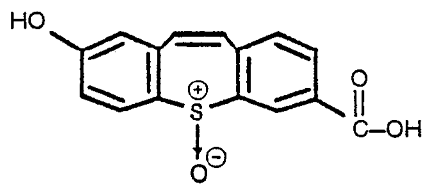

oder eine Verbindung, die ein pharmazeutisch annehmbares Salz davon ist.

2. 8-Fluordibenzo[b,f]thiepin-3-carbonsäure.

3. 8-Fluordibenzo[b,f]thiepin-3-carbonsäure-5-oxid.

4. Das R(—)-Isomer von 8-Fluordibenzo[b,f]thiepin-3-carbonsäure-5-oxid.

5. 8-Fluordibenzo[b,f]thiepin-3-carbonsäure-5,5-dioxid.

6. 8-Hydroxydibenzo[b,f]thiepin-3-carbonsäure-5-oxid.

7. Eine Zusammensetzung zur Behandlung unerwünschter kontraktiler Wirksamkeit von Prostaglandinen, bestehend im wesentlichen aus einem pharmazeutisch annehmbaren Träger, und einer therapeutisch wirksamen Menge einer in irgendeinem der Ansprüche 1 bis 6 beanspruchten Verbindung.

8. Eine Verbindung, wie sie in irgendeinem der Ansprüche 1 bis 6 beansprucht ist, für die Verwendung bei der Behandlung unerwünschter kontraktiler Wirksamkeit von Prostaglandinen in einem eine solche Behandlung benötigenden Wirt.

9. Die in Anspruch 4 beanspruchte Verbindung zur Verwendung bei der Behandlung asthmatischer Erkrankungen.

10. Ein Verfahren zur Herstellung einer Verbindung der Formel:

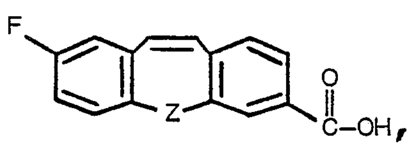

worin Z Thio, Sulfinyl order Sulfonyl ist, oder

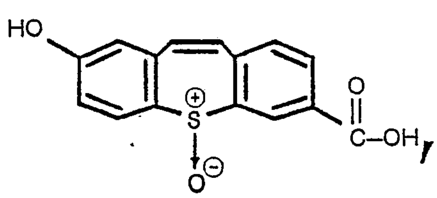

umfassend die Hydrolyse der entsprechenden Verbindung, die einen 3-Cyansubstituenten anstelle eines 3-Carboxylsubstituenten aufweist, mie einer Säure oder einer Base zur Erzeugung der gewünschten Verbindung.

11. Ein Verfahren zur Herstellung eines optischen Isomers von 8-Fluordibenzo[b,f]thiepin-3-carbonsäure-5-oxid, das in der R(—)-Konfiguration vorliegt, welches die Formel:

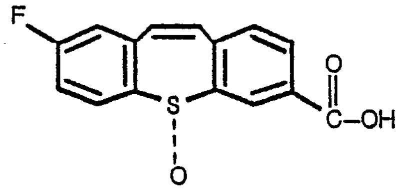

hat, umfassend die Regenerierung der Carbonsäure aus einer diastereoisomeren Verbindung der optisch aktiven Säure und einer optisch aktiven Form eines Esters oder eines Amids.

12. Ein Verfahren nach Anspruch 11, umfassend die Trennung eines Gemisches der Diastereoisomeren eines optisch aktiven Amids von 8-Fluordibenzo[b,f]thiepin-3-carbonsäure-5-oxid durch Chromatographie in eine weniger polare Fraktion eines Diastereoisomeren von S(+)-8-Fluordibenzo[b,f]thiepin-3-(—)-(N-$\alpha$-methylbenzyl)carboxamid-5-oxid und eine polarere Fraktion eines Diastereoisomeren von R(—)-8-Fluordibenzo[b,f]thiepin-3-(—)-(N-$\alpha$-methylbenzyl)-carboxamid-5-oxid, und das gesonderte Hydrolysieren der amiddiastereoisomeren R(—)-9-Fluordibenzo[b,f]thiepin-3-(—)-N-$\alpha$-methylbenzyl-carboxamid-5-oxid-Fraktionen zur Erzeugung des 8-Fluordibenzo[b,f]thiepin-3-carbonsäure-5-oxid R(—)-Isomers.

13. Ein Verfahren nach Anspruch 11, umfassend die Umwandlung der genannten Säure in das entsprechende Säurehalogenid, das Zusammenbringen des Säurehalogenids mit einem optisch aktiven Amin unter Bildung von 8-Fluordibenzo[b,f]thiepin-3-N-substituiertem-carboxamid-5-oxid als ein Gemisch der DL- und LL-Diastereoisomeren, die Trennung der diastereoisomeren Mischung durch Chromatographie in das weniger polare Diastereoisomer S(—)-8-Fluordibenzo[b,f]thiepin-3-(—)-N-substituiertes-carboxamid-5-oxid und das polarere Diastereoisomer R(—)-8-Fluordibenzo[b,f]thiepin-3-(—)-N-substituiertes-carboxamid-5-oxid, und das Unterwerfen des R(—)-8-Fluordibenzo[b,f]thiepin-3-(—)-N-substituierten-carboxamid-5-oxid einer sauren Hydrolyse unter Bildung von 8-Fluordibenzo[b,f]thiepin-3-carbonsäure-5-oxid R(—)-Isomer.

14. Ein Verfahren wie in Anspruch 11 beansprucht, worin das Amin L-$\alpha$-Methylbenzylamin ist.

**Revendications**

1. Un composé de formule:

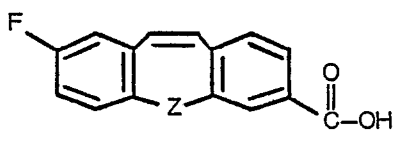

dans laquelle Z est un thio, un sulfinyle ou un sulfonyle; ou

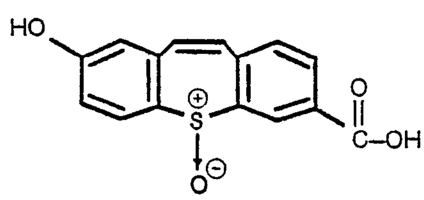

ou un composé qui en est un sel acceptable en pharmacie.

2. Acide 8-fluorodibenzo[b,f]thiépine-3-carboxylique.

3. Acide 8-fluorodibenzo[b,f]thiépine-3-carboxylique-5-oxyde.

4. L'isomère R(—) de l'acide 8-fluorodibenzo[b,f]thiépine-3-carboxylique-5-oxyde.

5. Acide 8-fluorodibenzo[b,f]thiépine-3-carboxylique-5,5-dioxyde.

6. Acide 8-fluorodibenzo[b,f]thiépine-3-carboxylique-5-oxyde.

7. Une composition pour le traitement d'une activité contractante indésirable des prostaglandines, constituée essentiellement d'un support acceptable en pharmacie et d'une quantité thérapeutique efficace d'un composé comme revendiqué dans l'une quelconque des revendications 1 à 6.

8. Un composé comme revendiqué dans l'une quelconque des revendications 1 à 6 pour l'emploi

dans le traitement d'une activité contractante indésirable des prostaglandines chez un hôte nécessitant un tel traitement.

9. Le composé revendiqué dans la revendication 4, pour l'emploi dans le traitement des maladies asthmatiques.

10. Un procédé pour préparer un composé de formule:

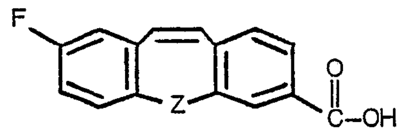

dans laquelle Z est un thio, un sulfinyle ou un sulfonyle, ou

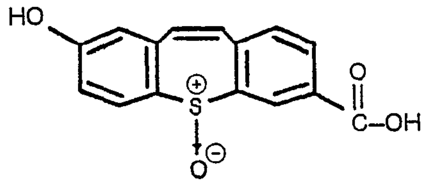

qui comprend l'hydrolyse du composé correspondant ayant un substituant 3-cyano au lieu du substituant 3-carboxy avec un acide ou une base pour produire le composé désiré.

11. Un procédé pour préparer un isomère optique d'acide 8-fluorodibenzo[b,f]thiépine-3-carboxylique-5-oxyde présent sous la configuration R(—) qui a pour formule:

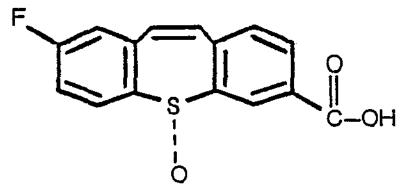

qui comprend la régénération de l'acide carboxylique à partir d'un composé diastéréo-isomère de l'acide optiquement actif et d'une forme optiquement active d'un ester ou d'un amide.

12. Un procédé selon la revendication 11, qui comprend la séparation d'un mélange de diastéréo-isomères d'un amide optiquement actif d'acide 8-fluorodibenzo[b,f]thiépine-3-carboxylique-5-oxyde par chromatographie en une fraction moins polaire d'un diastéréo-isomère de S(+)-8-fluorodibenzo[b,f]thiépine-3-(—)-(N-$\alpha$-méthylbenzyl)carboxamide-5-oxyde et une fraction plus polaire d'un diastéréo-isomère de R(+)-8-fluorodibenzo[b,f]thiépine-3-(—)-(N-$\alpha$-méthylbenzyl)carboxamide-5-oxyde et l'hydrolyse séparée des fractions de l'amide diastéréo-isomère R(+)-8-fluorodibenzo[b,f]thiépine-3-(—)-N-$\alpha$-méthylbenzylcarboxamide-5-oxyde pour produire l'isomère R(—) de l'acide 8-fluorodibenzo[b,f]thiépine-3-carboxylique-5-oxyde.

13. Un procédé selon la revendication 11, qui consiste à transformer ledit acide en l'halogénure d'acide correspondant, mettre l'halogénure d'acide en contact avec une amine optiquement active pour former le 8-fluorodibenzo[b,f]thiépine-3-(N-substitué)carboxamide-5-oxyde sous forme d'un mélange des diastéréo-isomères DL et LL, séparer le mélange diastéréo-isomère par chromatographie en le diastéréo-isomère le moins polaire, le S(—)-8-fluorodibenzo[b,f]thiépine-3-(—)-(N-substitué)carboxamide-5-oxyde, et le diastéréo-isomère le plus polaire, le R(—)-8-fluorodibenzo[b,f]thiépine-3-(—)-(N-substitué)carboxamide-5-oxyde, et soumettre le R-(—)-8-fluorodibenzo[b,f]thiépine-3-(—)-(N-substitué)carboxamide-5-oxyde à une hydrolyse acide pour former l'isomère R(—) de l'acide 8-fluorodibenzo[b,f]thiépine-3-carboxylique-5-oxyde.

14. Un procédé comme revendiqué dans la revendication 11, dans lequel l'amine est la L-$\alpha$-méthylbenzylamine.